(19)

Europäisches
Patentamt
European
Patent Office
Office européen
des brevets

(11) **EP 3 806 031 A1**

(12) **EUROPEAN PATENT APPLICATION**

(43) Date of publication:
**14.04.2021 Bulletin 2021/15**

(51) Int Cl.:
***G06T 7/00*** *(2017.01)*

(21) Application number: **19202004.8**

(22) Date of filing: **08.10.2019**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**KH MA MD TN**<br><br>(71) Applicant: **Koninklijke Philips N.V.**<br>**5656 AG Eindhoven (NL)**<br><br>(72) Inventors:<br>• **SCHRECKENBERG, Marcus**<br>**5656 AE Eindhoven (NL)**<br>• **BLITZ, Alexandra**<br>**5656 AE Eindhoven (NL)** | • **BLANKENHAGEN, Michael**<br>**5656 AE Eindhoven (NL)**<br>• **SCHUMMERS, Georg**<br>**5656 AE Eindhoven (NL)**<br>• **ROSSMANITH, Alexander**<br>**5656 AE Eindhoven (NL)**<br>• **ZOERNACK, Sabrina**<br>**5656 AE Eindhoven (NL)**<br>• **WIEBEL, Hendrik**<br>**5656 AE Eindhoven (NL)**<br>• **STAPF, Daniel**<br>**5656 AE Eindhoven (NL)**<br><br>(74) Representative: **Philips Intellectual Property & Standards**<br>**High Tech Campus 5**<br>**5656 AE Eindhoven (NL)** |

(54) **COMPUTER IMPLEMENTED METHOD FOR AUTOMATED ANALYSIS OF THE BIAS IN MEASUREMENTS PERFORMED ON MEDICAL IMAGES OF AN ANATOMICAL STRUCTURE**

(57)     The invention is directed to a computer implemented method for automated analysis of the bias in measurements performed on medical images of an anatomical structure. The method comprises the steps of: receiving a plurality of medical images, wherein the plurality of medical images includes at least a first plurality of medical images and a second plurality of medical images, and each plurality of medical images includes the same anatomical structure of the same or different patient(s), receiving, for the first plurality of medical images, first measurement data (RI) of a feature of the anatomical structure, wherein the first measurement data (RI) is obtained by a first user, determining, for the first plurality of medical images, a first baseline measurement data (C1) of the feature of the anatomical structure by means of an automated measurement tool (1), determining a first measurement bias (B1) for the first user based on the first measurement data (RI) and the first baseline measurement data (C1), receiving, for the second plurality of medical images, second measurement data (R2) of a feature of the anatomical structure, wherein the second measurement data (R2) is obtained by a second user, the second user being different from the first user, determining, for the second plurality of medical images, a second baseline measurement data (C2) of the feature of the anatomical structure by means of an automated measurement tool (1), determining a second measurement bias (B2) for the second user based on the second measurement data (R2) and the second baseline measurement data (C2), and comparing the first measurement bias (B1) with the second measurement bias (B2).

Fig. 3

**Description**

FIELD OF THE INVENTION

The present invention relates to a computer-implemented method for automated analysis of a user-specific bias in measurements performed on medical images of an anatomical structure, a computer program, and an automated measurement tool configured to perform the method.

BACKGROUND OF THE INVENTION

Medical ultrasound measurements are used in a wide field of medical treatment. For example, echocardiographic measurements are typically used as an indirect method to assess the dimensions and the function of a human's or animal's anatomical structure, specifically of the human's heart. However, measurement results are subject to inter-observer and intra-observer discrepancies. The Bland-Altman analysis is a common method used in the prior art for determining both the variability and the bias between two different observers (such as users or physicians). For accreditation purposes, labs that perform measurements (specifically echocardiography labs) have to undergo a standardization procedure in order to comply with quality guidelines. Further, this accreditation includes: Interpretive Quality Review (Physician Interpretation Variability). The facility must evaluate the quality and accuracy of the interpretation based on the acquired images. A minimum of two cases per modality per quarter must be evaluated for the quality and accuracy of the interpretation of the acquired images. The cases must represent as many physicians as possible. Differences in interpretation must be reconciled to achieve uniform examination interpretation. In daily clinical routine, this translates into an additional manual and organizational workload. This is even worse in case of long-term clinical studies in which the measurement observers often change during the realization of the study. Therefore, there is a problem of inter-observer variability and consensus training.

It would, therefore, be desirable to have an independent detection and measurement system with standardized performance and uniform bias, simplifying the adherence to quality guidelines and usable to calibrate and/or normalize measurement results of different users.

Christian Knackstedt discloses in "Fully Automated Versus Standard Tracking of Left Ventricular Ejection Fraction" in JOURNAL OF THE AMERICAN COLLEGE OF CARDIOLOGY; VOL. 66, NO. 13, 2015 a comparison of measurement results made by a human with measurement results made by a machine, wherein the left ventricular volume and longitudinal strain measurements were assessed.

US 2018150598 A1 discloses to compare the anatomical measurement acquired using machine learning with a predetermined threshold and to generate advisory information to adjust the measurement.

Vikram Chalana focuses in "A Methodology for Evaluation of Boundary Detection Algorithms on Medical Images" IEEE TRANSACTIONS ON MEDICAL IMAGING, VOL. 16, NO. 5, OCTOBER 1997 on the comparison of differences in contouring. That is, it compares computer-generated boundaries to multiple observer-created boundaries and calculates a Cohen Kappa-coefficient of agreement between two observers.

US 2014341449 A1 discloses to reduce inter observer and intra observer variability in contouring images by verifying the consistency using consensus contours generated by machine learning. Further, according to US 2014341449 A1 a degree of deviation of the user created contour from the consensus contour is evaluated.

Harold L. Kunde discloses in "Measurement of Observer Agreement" in Radiology 2003; 228:303-308 a statistical measure for measuring agreement between two readers utilizing Cohen Kappa which is used to measure categorical answers (positive vs. negative).

As outlined above, the echocardiographic measurements are one example of an indirect method to assess the dimensions and the function of the human heart. Between the measurements of two observers (inter observer) or even between two measurement instances of the same observer (intra observer), the results are bound to vary. A common method for the analysis of both the variability (i.e. the margin of error, or 95% confidence interval, of the difference between two observers) and the bias (i.e. the systematic error, or the mean difference between two observers) is the Bland-Altman-Analysis. The Bland-Altman method can be used to evaluate the agreement between two series of measured values. In particular, it provides information on the influence of the height of the measured values on the size of the deviations/differences. For this purpose, the differences (measurement differences) are represented as a function of the averages (measured value height) in a scatter diagram (Bland-Altman plot).

In addition to a regression line with confidence interval, the zero line (difference=0) as well as the mean difference (also with confidence interval) and sometimes also the limits of agreement are usually displayed. This information can be used to evaluate the degree of redundant dimensioning.

OBJECT OF THE INVENTION

It is therefore an object of the present invention to provide a computer implemented method for automated analysis of the bias in measurements performed on medical images of an anatomical structure, in which the quality guidelines are met while the manual and organizational workload are reduced. It is also an object of the present invention to provide a respective computer program, and an automated measurement tool for executing the method.

SUMMARY OF THE INVENTION

To better address one or more of the above-identified concerns, in a first aspect of the invention a computer implemented method for automated analysis of the bias in measurements performed on medical images of an anatomical structure is presented in claim 1. Useful embodiments are set out in the dependent claims.

In accordance with this first aspect, a computer implemented method for automated analysis of the bias in measurements performed on medical images of an anatomical structure comprises the steps of:

> receiving a plurality of medical images, wherein the plurality of medical images includes at least a first plurality of medical images and a second plurality of medical images, and each plurality of medical images includes the same anatomical structure of the same or different patient(s),
> receiving, for the first plurality of medical images, first measurement data of a feature of the anatomical structure, wherein the first measurement data is obtained by a first user,
> determining, for the first plurality of medical images, a first baseline measurement data of the feature of the anatomical structure by means of an automated measurement tool,
> determining a first measurement bias for the first user based on the first measurement data and the first baseline measurement data,
> receiving, for the second plurality of medical images, second measurement data of a feature of the anatomical structure, wherein the second measurement data is obtained by a second user, the second user being different from the first user,
> determining, for the second plurality of medical images, a second baseline measurement data of the feature of the anatomical structure by means of an automated measurement tool,
> determining a second measurement bias for the second user based on the second measurement data and the second baseline measurement data, and
> comparing the first measurement bias with the second measurement bias.

Accordingly, in an embodiment, the invention provides a method in which the same medical images are analysed by both a human user and an automated measurement tool, and the results are compared with each other to obtain a measurement bias for the human user. Thus, two different measurement instances or observes (human and machine) may be used on the same medical images, and a bias is determined between them. This bias may then be used to estimate the bias between different humans, such as the first and the second user. Generally, a bias may be a measure of how much the measurement data differs from the true underlying quantitative parameter being estimated. For example, a measured value is biased if it is determined in such a way that it is systematically different from the population parameter being estimated. Moreover, the bias may be a discrepancy metric (i.e. deviation metric) or a margin of error, in other words, the systematic error or the mean difference between two users (i.e. observers).

Particularly, systematic errors may change during an experiment (i.e. drift). That is, measurement results may indicate trends with time rather than varying randomly about a mean. Drift is evident if a measurement of a constant quantity is repeated several times and the measurements drift one way during the experiment. If the next measurement is higher than the previous measurement as may occur if an instrument becomes warmer during the experiment, for example, then the measured quantity is variable and it is possible to detect a drift by checking the zero reading during the experiment as well as at the start of the experiment. On the other hand, in case there is no pattern in a series of repeated measurements, the presence of fixed systematic errors can only be found if the measurements are checked, either by measuring a known quantity or by comparing the readings (i.e. the measurement results) with readings made using a different apparatus, known to be more stable (i.e. providing measurement results without a drift). Specifically, the present invention is particularly useful in the latter cases. That is, according to the present invention the baseline measurement data (to be described below) is attained by an automated measurement tool (which might be considered as said different apparatus). Further, the automated measurement tool may be also referred to as a baseline machine. In other words, the present invention provides a calibration/normalization tool in which the measurement bias of a given user may be associated to the systematic (and subjective) difference that said user generates when doing a predetermined measurement on the anatomical structure included in the plurality of medical images.

Generally, the first and second measurement bias may be an absolute measurement bias or a relative measurement bias. In more detail, the first and second absolute measurement bias for the first and second users are preferably determined by calculating the difference between the first/second measurement data and the first/second baseline measurement data, respectively, i.e. by subtracting one from the other. On the other hand, the relative measurement bias may be defined by being normalized with respect to the measurement data (i.e. to the kind of measurement). In other words, the relative measurement bias may be determined based on a relative difference of the measurement data and the baseline measurement data instead of the difference of the measurement data and the baseline measurement data (i.e. as compared to the absolute measurement bias). Specifically, the relative measurement bias may be determined by the following equation:

$$B_{x,rel} = \frac{(Rx - Cx)}{\frac{1}{2}(Rx + Cx)}$$

Wherein

$B_{x,\,rel}$     represents the relative measurement bias,
Rx represents the measurement data,
Cx represents the baseline measurement data.

Accordingly, the absolute bias may have a functional relationship to data and/or physical properties of the measurement data on which it is based. Specifically, the absolute bias may have a functional relationship to properties of the modality used in creating the medical images, to properties of the measurement tool used in creating the measurement data, to circumstances present during the measurement and/or during the creating of the medical images and/or to absolute values of the measurement data. Therefore, the absolute measurement bias may not be readily compared with other absolute measurement biases that are obtained based on different measurement data (e.g. on measurement data that includes a measurement of a different feature of the anatomical structure). The relative bias may be independent from such functional relationships. As a result, the relative measurement bias may be compared with other relative measurement biases based on different measurement data.

Therefore, according to another embodiment of the present invention, for the measurement bias, a relative difference between the measurement data and baseline measurement data may be determined. That is, by determining the relative difference between the measurement data and baseline measurement data, the relative bias may be determined (for example as outlined above). The relative bias is especially useful in the following case: If a relatively small dimension is measured (for example the thickness of a septum of a heart), a 10 % deviation from the baseline measurement data results in a relatively small absolute bias. On the other hand, if a relatively large dimension is measured (for example the diameter of the left ventricle of the heart), a 10 % deviation results in a relatively high absolute bias. As a result, although both measurements have the same percentage deviation, the absolute bias for each measurement differs. Therefore, in order to remedy this deviation of the absolute bias, the relative bias may be determined. As a result, relative measurement biases may be compared with each other independent of the measurement data on which the measurement biases are based. In other words, independent of the feature of the anatomical structure which is measured, for example.

In the following description, for convenience of explanation, the absolute bias and the relative bias are simply referred to as bias. However, the term bias always refers to both the absolute bias and the relative bias.

Further, a per-user bias may be determined. The per-user bias may be represented by a mean value of all measurement biases of a specific user.

Medical images may be generated by any medical imaging modality, such as ultrasound, in particular echocardiography, or MRI (Magnetic Resonance Imaging), X-Ray Imaging, CT (Computed Tomography), PET (Positron Emission Tomography), and so on. When using ultrasound, the images may also be obtained by Colour Doppler, Tissue Doppler, Continuous Wave Doppler, Pulsed Wave Doppler or M-Mode, i.e., there is a variety of acquisition modes that may be the basis for manual or automated measurements. The medical images may be 2D images or 3D images. The medical images (or one set of medical images) may be one or several or all of a sequence of frames of 2D or 3D images that constitute a 4D image, wherein time is the fourth dimension.

The anatomical structure may be any anatomical structure of the human or animal body, such as a limb, an organ or a blood vessel, for example the heart, the liver, or a fetus. The same structure means that if the first plurality of first medical images includes the heart as the anatomical structure, the second plurality of medical images also includes the heart as the anatomical structure.

The medical images may be derived from the same patient or from different patients, that is, the medical images may be also derived from the same patient but at different points of time. Further, the method or modality used for obtaining the medical images may be different from one image to the other. For example, the first plurality of medical images may be obtained using an echocardiography system, wherein the second plurality of medical images may be obtained using an MR system, for example. Further, the first plurality of medical images and the second plurality of medical images may not necessarily have to be received at the same location. That is, the inventive method may be partly executed at different locations, i.e. distributed over several locations. Preferably, only the step of comparing the first measurement bias with the second measurement bias may be centralized (i.e. may be executed at one location), whereas the other previous method steps may be executed elsewhere. Specifically, the method step of receiving, for the first plurality of medical images, first measurement data of a feature of the anatomical structure, wherein the first measurement data is obtained by a first user, determining, for the first plurality of medical images, a first baseline measurement data of the feature of the anatomical structure by means of an automated measurement tool and determining a first measurement bias for the first user based on the first measurement data and the first baseline measurement data may be executed at one location. Similarly, the corresponding method steps related to the second plurality of medical images may be executed at a different location. As a result, only the first measurement bias and the second measurement bias have to be transmitted for being compared with each other. In other words, the first medical images may be received and processed (i.e. receiving the first measurement data and determining the first baseline measurement data) at a core lab in Europe while the second medical images may be received and processed (i.e. receiving the second measurement data and determining the second baseline measurement data) at a core lab in the US. Further, the comparison of the first measurement bias and the second measurement bias may be performed at a different location or at any of the above locations. That is, only the first and second measurement biases may have to be transmitted for comparing them. The transmission may be performed via the internet, for example.

Naturally, the invention may be extended to a third and further pluralities of medical images, and related third and further measurement data and baseline measurement data, to determine a third and further measurement biases of a third and further users.

The measurement data may represent a physical parameter, e.g. a dimension, of the anatomical structure that is to be analysed, in particular a length, area or volume. It may also be a flow rate, time period or other physical parameter that can be measured from medical images. In more detail, the measurement data may include a diameter and/or a circumference of a heart valve such as the mitral valve and/or the aortic valve. The measurement data may be manually taken by at least one user. In more detail, the measurement data may be attained by the user by tracing a structure in the medical image, for example. Further, the measurement data may be additionally or alternatively generated by the user by drawing a 3D free hand line in a 3D data set. Further, the measurement data may include a contour of an anatomical structure.

The acquisition of the medical images may have been performed by the same user, who will also carry out the (manual) measurements to obtain the measurement data. The examination may also be performed by other clinical staff, as is usually the case in MR examinations.

In an embodiment, each plurality of medical images includes one or several sets of medical images, wherein each set of medical images is obtained during one examination session from one patient. Preferably, one measurement data of a feature of the anatomical structure and one baseline measurement data of the feature of the anatomical structure is obtained from each set of medical images, and preferably each plurality of medical images includes several sets. Usually, each plurality of medical images will thus include sets of medical images from a plurality of patients. However, a plurality of medical images may also include two or more sets of medical images acquired at different examination sessions from the same patient, as will be the case in longitudinal studies. For example, one set of medical images may include all ultrasound images obtained during one examination session from the heart of a patient, thus including a number of 2D and/or 3D images from various viewing angles and with different field of views.

The user may be any person working in the field of medical image analysis such as a doctor, a nurse, an employee in a measurement lab and so on. The first and second user are usually different persons. Possibly, the first and second user may be the same person that creates the measurement data at different times or with different measurement techniques.

In an embodiment, the first (second) measurement data of the feature of the anatomical structure are created by the first (second) user performing manual measurements on one or several of the first (second) plurality of images, for example by measuring a physical parameter, e.g. a length or area. Such measurement will usually be carried out manually, i.e. requiring user intervention, but with the aid of a computer, e.g. by the user clicking on specific landmarks in the medical image displayed on a computer screen, and the distance between the landmarks being calculated automatically by the computer. Generally, the measurement data may be obtained using a computer-implemented measurement technique requiring user intervention, e.g. such that the user is responsible for the correctness of the measurement data.

Receiving may mean that the measurement data is received in real time, that is, directly after the measurement data is obtained. Alternatively, the measurement data may be received from a storage, that is, previously generated and stored measurement data may be analysed in hindsight. As a result, older studies performed in the past, may be newly analysed and thus further processed using the inventive method. Further, the measurement data may be received by the automated measurement tool. In addition, the first measurement data may be received by a first automated measurement tool and the second measurement data may be received by a second automated measurement tool, wherein the first and second automated measurement tool may be separated tools.

The first and second baseline measurement data of the feature of the anatomical structure is obtained automatically, i.e. without user intervention, by the automated measurement tool. Optionally, the user may select a particular medical image, on which the baseline measurement is to be taken, e.g. one medical image from a set of medical images obtained during one examination session. Preferably, the baseline measurement data are obtained by a computer-implemented algorithm. Such algorithm may be a classical algorithm, for example an algorithm using techniques such as feature tracking, segmentation techniques (e.g. thresholding, region growing, pixel histogram analysis etc.). Alternatively, it may be a machine-learning algorithm, in particular, an algorithm based on deep-learning techniques and including one or several artificial neural networks. Such artificial neural networks may be trained on images from a reference database and measurement data obtained by an expert user, optionally verified by a second user, to minimize any errors.

Baseline measurement data are determined by means of the automated measurement tool, which may include all standard measurements, that are usually performed on the anatomical structure. Further, the automated measurement tool may determine the baseline measurement data by an algorithm (included in the automated measurement tool) including rules based on a reference database. In more detail, the reference database may be created by at least one expert (human) and the algorithm may be configured to measure bias free and/or with a constant bias with respect to said reference database. In addition, the results of the automated measurement tool are reproducible, that is, in case the same measurement data are available, the same results (i.e. baseline measurement data) are obtained by the automated measurement tool. As above with respect to the step of receiving the measurement data, the first and second baseline measurement data may be determined by the first and second automated measurement tool.

The automated measurement tool may be operated in the background of a currently executed application so as to determine the baseline measurement data hidden from the user. That is, the user must not necessarily notice that the baseline measurement data is currently being determined. The automated measurement tool is preferably consistent in its results, that is, it produces the same results (output), for the same image or sequence of images (input). In particular, the standard measurements may include all important measurements performed on a specific anatomical structure. In case the anatomical structure is the heart, the important measurements include the measurement of the diameter of the heart valve, the contour of a heart chamber, for example. The automated measurement tool may be connected to a PACS (Picture Archiving and Communication System) of a clinic or a lab, for example. Accordingly, all medical images that are introduced in such PACS may be considered by the automated measurement tool. As a result, a plurality of measurement biases may be automatically determined by the automated measurement tool. Further, the automated measurement tool may be included in a modality for creating medical images and/or for executing measurements on medical images. In more detail, the measurement bias may be determined directly within the respective modality (i.e. in situ). Therefore, only the measurement bias may have to be transmitted for the purpose of comparing it with further measurement biases. Moreover, even the step of comparing two measurement biases with each other may be executed within the modality and the result of such comparison may be outputted.

In the case where several measurement data of the anatomical structure (e.g. the diameter of the left ventricle) measured by the first user are received, the baseline measurement data may be determined by the automated measurement tool for each of the measurement data. Then the difference between each measurement data and the baseline measurement data may be determined, so there is a baseline measurement data for each measurement data. The first measurement bias may then be determined as the average or mean of each of the baseline measurement data for the first user. Thus, the first measurement bias may be a representation of the systematic difference between measurements performed by the first user, compared to the baseline measurement data obtained. The same holds for the second measurement bias.

Finally, the first measurement bias is compared with the second measurement bias, for example by subtracting one from the other, or by simply reviewing the measurement bias of each user. The method of the invention may be extended to further users, e.g. by repeating the steps for a third, fourth and further users.

In case of both longitudinal and multi-centre studies, the bias comparison results can be used to normalize/calibrate the measurement values between users.

According to the above embodiment of the present invention, an automated analysis of two or more observers (even on differing patients) may be executed. Through a bias comparison of every user (in more detail, of every user's measurement data) with the automated measurement tool, the mean bias between the two users can be calculated for all patients. This result may be used to give feedback to all involved physicians (users) for routine clinical procedures and/or clinical long-term studies without the need to measure identical studies. For this functionality, the medical images measured by the user may be used to perform the analysis using the automated measurement tool in order to minimize error sources between the user and the automated measurement tool. In case of clinical long-term studies (both longitudinal and multi-centre studies), the bias comparison results can be used to normalize the measurement values between observers in case of the clinical study scenario. Through applying the measurements on all available views, more measurements than in the manual case may be generated, thus leading to a more stable system with less statistical noise in the analysis. Therefore, the reduction of manual and organizational workload to ensure a high quality can be ensured. Using the above method, measurement results of users may be normalized and/or calibrated. The method is applicable worldwide, that is, it is not locally bounded. Further, the different users do not have to be at the same location. Therefore, the method provides an increased flexibility in its application.

According to a further embodiment of the present invention, the method may further comprise the step of determining a global bias represented by a mean value of all available measurement biases, and comparing the global bias with one of the measurement biases.

The global bias may be obtained by transmitting locally determined measurement biases to a centralized institution. At the centralized institution, the global bias may be determined by calculating the average or mean value of all locally determined measurement biases. For example, measurement biases may be transmitted to the centralized institution from PACS and/or modalities including the automated measurement tool. Further, in order to determine the global bias, biases may be used that are already a mean value of a plurality of biases. Further, the automated measurement tool could have a systematic bias against all human observers (i.e. all users). By determining the global bias, this systematic bias may be determined. In addition, each user may compare his/her own bias with the global bias in order to assess his own bias. Further, a measurement bias for standard values for specific measurements of anatomical structures (e.g. as published in several guidelines) may be determined and compared with the global bias. Further, a user may compare his/her own bias with the global bias. Finally, the user may compare both comparison results in order to evaluate himself/herself with respect to the standard values. Thereby, each user may verify himself/herself.

According to a further preferred embodiment of the present invention, the first measurement data may be determined based on one specific medical image out of the first plurality of medical images and the first baseline measurement data may be determined based on the same specific medical image. Further, the second measurement data may be determined based on one specific medical image out of the second plurality of medical images and the second baseline measurement data may be determined based on the same specific medical image.

Generally, the user that performs a measurement on an anatomical structure in order to obtain measurement data, may choose a specific image (i.e. a specific view of the anatomical structure) which promises the most accurate measurement among the set of medical images. Further, the chosen image may be then used to perform the measurement. In order to minimize error sources between human (i.e. the user) and machine (i.e. the automated measurement tool) the image chosen by the user may be also used to perform the analysis by the automated measurement tool. According to this, the process is highly efficient and needs fewer computing resources and less time. However, all available images of the plurality or of each set of medical images may be analysed by the automated measurement tool. As a result, more measurements than in the manual case may be generated, thus leading to a more stable system with less statistical noise in the analysis.

In an embodiment, each measurement data and baseline measurement data is determined based on the same specific medical image from each set of medical images acquired during one examination session of a patient.

According to a preferred embodiment of the present invention, the measurement bias may be continuously updated once new measurement data is available, wherein all available measurement data are used to update the measurement bias, or wherein all available measurement data obtained during a pre-determined past period of time are used to update the measurement bias.

In other words, updated may mean that the bias is newly determined based on new available measurement data. Further, continuously may mean that the measurement bias is frequently updated. Moreover, update may mean that the old measurement bias is replaced by a newly determined measurement bias. This update procedure may be executed automatically without any specific actuation by the user. Further, the update may be executed such that the user does not notice that there is an update going on. The update may be performed by calculating a new average, which incorporates the new difference between new baseline measurement data and measurement data. The update is carried out as often as possible once new measurement data is available. In some cases, where the method steps are executed at different locations, there may be a delay in updating the measurement bias because no connection for transmitting of the newly determined measurement bias or measurement data exits. Then, the update may be carried out if the newly determined measurement bias or measurement data could be transmitted.

In an embodiment, the measurement biases for each user may be based on measurement data obtained during a pre-determined past period, for example the past one to three months. Older measurement data thus do not contribute anymore. Rather, at any point in time, the measurement bias of a user represents his average bias in all measurements performed during the pre-determined past period. That is, the measurement bias may be updated based on measurement data created in a pre-defined period of time, so as to update the measurement bias using a moving average of the measurement biases in said period of time. Alternatively, all available measurement data are considered by updating the measurement bias. That is, the newly obtained measurement data (and thus the newly determined measurement bias) may be considered in addition to all old measurement bias/measurement data.

According to the above embodiment, the measurement bias may be automatically adapted to new situations, that is, to an increased/decreased measuring accuracy of the user (e.g. after the user has attended a training).

According to a further preferred embodiment of the present invention, the method may further comprise the step of determining an institution wide bias represented by a mean value of all measurement biases of the first, second and further users belonging to one predetermined group or institution.

In other words, the institution wide bias may be a mean value of all measurement biases of the users belonging to said institution, wherein the institution may be a core lab, clinic, research team etc. However, the institution wide bias may be the mean bias of a group of users constituted by a predetermined number of users. Mean value may be the sum of all values divided by the number of values.

As a result, it may be easy to objectively evaluate the bias of a working group without the need to evaluate each independent bias of each user. Further, different groups or institutions may be easily compared with each other.

According to a preferred embodiment of the present invention, the method may further comprise the step of comparing the institution wide bias with one of the measurement biases so as to obtain a per-user bias relative to the institution wide bias.

In other words, the per-user bias may be the individual bias of a user. Further, the institution wide bias may be the same as outlined above. Accordingly, each user may be evaluated with respect to the mean value of all user's measurement biases belonging to the institution. Further, the evaluation is facilitated in that it is easily driveable which user improves the institution wide bias and which user degrades the institution wide bias.

According to a preferred embodiment of the present invention, the step of comparing the institution wide bias with one of the measurement biases may include: determining a difference by subtracting each of the measurement biases by the institution wide bias so as to determine the difference for each user belonging to the same group or institution, and determining the user or users having the highest difference(s) among all users.

In other words, an additional value is calculated (i.e. the difference) representing the difference between the mean value of the institution wide bias and the individual per-user measurement bias. Accordingly, the determination which user has to improve his/her measurement accuracy is facilitated. Further, the above embodiment allows to create an agenda on which user needs to increase his/her accuracy in order to improve the institution wide bias to a predetermined level.

According to a further embodiment of the present invention, the determination of at least the first baseline measurement data and the second baseline measurement data may take place at different locations. In other words, the measurement data may be further processed (including the determination of the baseline measurement data and the determination of the measurement bias) at the same location where the measurement data were obtained ore received, for example. That is, the automated measurement tool may be present at the respective location where the measurement data is obtained or received so as to locally determine the baseline measurement data. In addition, the measurement bias may be also locally determined at the same location where the measurement data is obtained or received. As a result, only the baseline measurement data and/or the measurement bias have to be transmitted for the further processes.

According to a further preferred embodiment the automated measurement tool may use artificial intelligence for determining the baseline measurement. That is, the automated measurement tool may operate in a rule-based manner. For example, the automated measurement tool may automatically perform a grey value analysis of the medical image so as to determine boundaries between tissue and blood flow. Further, the automated measurement tool may perform an image analysis of the medical image so as to find specific geometrical objects. Based on those analyses, the measurement tool may identify the feature of the anatomical structure that is to be measured. Subsequently, the automated measurement tool may perform the measurement so as to obtain the baseline measurement data.

According to a further preferred embodiment, the automated measurement tool may use machine learning for determining the baseline measurement, wherein the automated measurement tool may be trained by a reference data base including reference images of the anatomical structure and measurement data obtained by at least one expert user for said reference medical images.

The reference data base includes examples of standard measurements (i.e. example measurements dates) generated by experts. In particular, the standard measurements may include all important measurements performed on a specific anatomical structure. As a result, the automated measurement tool may measure an arbitrary anatomical structure based on the example dates included in the reference data base. Further, the automated measurement tool may determine the baseline measurement data such that the bias with respect to the reference data base is minimized and/or constant. Further, the determination of the baseline measurement data may be reproducible. That is, if the same input data (i.e. medical images) are inputted, the automated measurement tool outputs the same output (i.e. the baseline measurement data). In other words, the results determined by the automated measurement tool may be reproducible. Therefore, the automated measurement tool may be suited for a reliable baseline measurement.

According to a further preferred embodiment of the present invention, the automated measurement tool may use an artificial neural network for determining the baseline measurement data.

In more detail, artificial neural networks (also referred as connectionist systems) may be computing systems that "learn" to perform tasks by considering examples, generally without being programmed with task-specific rules. For example, in image recognition, the artificial neural networks might learn to identify images that contain a specific content by analysing example images that have been manually labelled to contain the specific content and using the results to identify the specific content in other images. The artificial neural networks do this without any prior knowledge of the specific content. Instead, they automatically generate identifying characteristics from the examples that they process. In the same way, the artificial neural networks according to the present embodiment analyse the reference database. Specifically, the reference database includes examples of standard measurements performed by experienced experts having a very low inter-observer bias. As a result, the artificial neural networks may perform the measurement on unknown (i.e. newly obtained medical images) having substantially the same bias as the experienced experts. Further, the artificial neural networks may analyse all images of the plurality of medical images in order to generate a mean bias which is less susceptible to measurement errors that have occurred in a specific image. Further the artificial neural networks may be based on a collection of connected units or nodes called artificial neurons. Each connection, like the synapses in a biological brain, can transmit a signal to other neurons. An artificial neuron that receives a signal then processes it and can signal neurons connected to it.

Accordingly, the mean value (i.e. the baseline measurement data) outputted by analysing the plurality of medical images of the same anatomical structure is less susceptible to measurement errors that have occurred in a specific image, that is, the baseline measurement data needs to be accurate only in the mean value, whereas a single outlier is innocuous.

According to a preferred embodiment of the present invention, the measurement data may include at least one of a dimension such as a length, area or volume, or volume flow, velocity of blood and velocity of tissue. The present invention may be applied to a wide field of measurement data that may be obtained from medical images. Further examples for measurement data are shown in "The American Society of Echocardiography Recommendations for cardiac chamber Quantification in Adults: A Quick Reference Guide from the Ase Workflow and Lab Management Task Force", in "Recommendations for Cardiac Chamber Quantification by Echocardiography in Adults: An Update from the American Society of Echocardiography and the European Association of Cardiovascular Imaging" published in the Journal of the American Society of Echocardiography Volume 28 by Lang et al and in "Recommendations for the Evaluation of Left Ventricular Diastolic Function by Echocardiography: An Update from the American Society of Echocardiography and the European Association of Cardiovascular Imaging" published in the Journal of the American Society of Echocardiography in April 2016 by Nagueh et al.. All above citations are incorporated herein by reference. In addition, the measurement data may include a dimension of a fetus, such as at least one of crown-rump-length, nuchal translucency, biparietal diameter, femur length, humerus length, head circumference, abdominal circumference. Further, the measurement data may include Doppler flow profiles, cardiac valve diameter and/or ventricle dimensions.

According to a further preferred embodiment of the present invention, for the measurement bias, a new measurement bias and an old measurement bias may be determined, wherein the new measurement bias may be determined when a predetermined period of time has elapsed since the old measurement bias was determined, or the new measurement bias may be determined when a predetermined amount of new measurement data are available since the old measurement bias was determined, and wherein the new measurement bias may be compared with the old measurement bias so as to obtain a drift in the measurement bias.

In other words, the old measurement bias may be a measurement bias that is determined previous to the new measurement bias. Further, the two measurement biases may be compared with each other, wherein each of the measurement biases may be separated from the other one either by a predetermine period of time (e.g. one to three months) or by a predetermined amount of measurement data (e.g. 10 to 100 measurement data, preferably 20 to 50) that are obtained since the previous measurement bias was determined. As a result, an analysis of trends may be performed so as to determine in which way the measurement bias changes from the old measurement bias to the new measurement bias. Accordingly, a drift in relation to a previously defined consensus may be recognized. That is, the consensus may be defined at the same time as the old measurement bias was determined (e.g. at the beginning of the collection of data). This knowledge may be in particular interesting for core labs and for long-term studies.

According to another aspect, the invention provides a computer program comprising program code instructions, which, when executed by a processor, enables the processor to carry out the inventive method. The computer program may be in any code, in particular a code suited for computer vision applications, in particular for measurement purposes of features of anatomical structures in medical images. Specifically, computer vision (also referred as machine vision) is a technology used to provide imaging-based automatic inspection and analysis. Therefore, this technology is in particular useful for use in the automated measurement tool.

In a further aspect, the invention is directed to a computer-readable medium comprising an above defined computer program. The computer-readable medium may be any digital data storage device, such as a USB-stick, hard disk, CD-ROM, SD card or SSD card. Naturally, the computer program need not be stored on such a computer-readable medium to be supplied to the costumers, but may be downloaded via the internet.

According to a further aspect, the invention is directed to an automated measurement tool configured to execute the method according to the above outlined, wherein the automated measurement tool includes an interface configured to receive a medical image generated by an imaging system and to output results determined by executing the inventive method. Any features or useful embodiments described in connection with the inventive method also apply to the automated measurement tool. Further, the automated measurement tool may be included in an imaging modality for obtaining medical images and or in a measuring device for creating measurement data based on the medical images. In particular, the measurement data may be transferred to the interface of the automated measurement tool as an input to the automated measurement tool. The transfer may be established via cable, near field connection, Wi-Fi, W-Lan or other suitable connections. After processing the above method steps, at least one of the results may be outputted via the interface of the automated measurement tool. Accordingly, the automated measuring tool may be locally applied in locations where measurement data are obtained or received. Alternatively or additionally, the measurement tool may be connected to a database (such as a PACS) storing a plurality of measurement data. Further, the measurement tool may be separately provided like a server connected to several clients (e.g. imaging modalities, measuring modalities, data storage, separate users, institutions, groups, etc.). Moreover, the method steps may be executed by more than one automated measurement tool. That is, one automated measurement tool may determine the measurement bias and another automated measurement tool may update said measurement bias or may determine the global bias, for example. Further, the automated measurement tools may be present at different locations.

In case the automated measurement tool is implemented in an imaging system, the medical images may be internally transmitted from the imaging system to the automated measurement tool and the measurement bias may be calculated in situ within the system itself by the automated measurement tool. As a result, merely the measurement bias has to be transmitted (outputted from the automated measurement tool) for further processing. Therefore, the amount of data that is to be transmitted may be significantly reduced and it is not necessary to establish a high capacity transmission line for the purpose evaluating the measurement bias. Further, the automated measurement toll may be applied on a workstation as well as in PACS (Picture Archiving and Communication System) systems.

In a preferred embodiment, the imaging system may be an ultrasound system.

SHORT DESCRIPTION OF THE FIGURES

Useful embodiments of the invention shall now be described with reference to the attached figures. Similar elements or features are designated with the same reference signs in the figures. In the figures:

Fig. 1 shows a schematic cross-section through a human heart (4-chamber view);
Fig. 2 is a diagram schematically showing an automated measurement tool according to an embodiment of the present invention;
Fig. 3 is another schematic diagram showing the measuring results of the user with respect to the baseline measurement data obtained by the automated measurement tool according to the embodiment of the present invention;

Fig. 4 is a flow chart that illustrates the steps of the method according to an embodiment of the present invention;
Fig. 5 shows a system including the automated measurement tool according to an embodiment of the present invention.

DESCRIPTION OF EMBODIMENTS

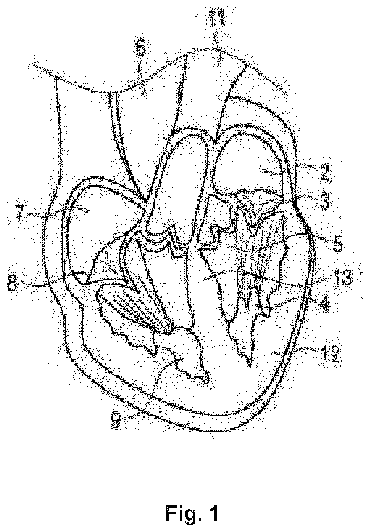

In order to better visualise the preferred application of the inventive method for automated analysis of the bias in measurements performed on medical images of an anatomical structure, FIG. 1 illustrates the structure of the human heart as an example of the anatomical structure. The blood coming from the lungs flows into the left atrium 2 and from there through the mitral valve 3 into the left ventricle 4. From there, it is pumped through the aortic valve 5 into the aorta 6. This part is also termed left ventricular outflow tract (LVOT). The blood coming from the body flows into the right atrium 7 and is pumped through the tricuspid valve 8 into the right ventricle 9. From there, it is pumped through the pulmonary valve into the pulmonary artery 11. Heart wall 12 is made of muscular tissue surrounding the heart chambers 2, 4, 7 and 9. The left and right ventricles are separated by the septum 13. It is evident from FIG. 1 that the heart has a complex shape, and in addition is constantly moving with the heartbeat, i.e. it is a dynamic anatomical structure. Such an anatomical structure, specifically the human's heart is recorded using medical imaging systems like echocardiography systems, ultra sound systems, MR, CT, etc. in order to attain medical images. The medical images are subsequently used to measure features of the anatomical structure, for example, during routine clinical processes and during clinical long-term studies. Due to the complexity of the anatomical structures, measuring of shapes, contours, distances and/or areas such as of the mitral valve 3 in order to analyse the feature of the anatomical structure is difficult and prone to individual errors. Generally, specified facilities execute such measurements in order to generate measurement data. That is, the measurement data may be generated in clinics or other medical facilities. For accreditation purposes, these facilities (e.g. echocardiography labs) have to undergo a standardization procedure (as outlined above in the introductory part).

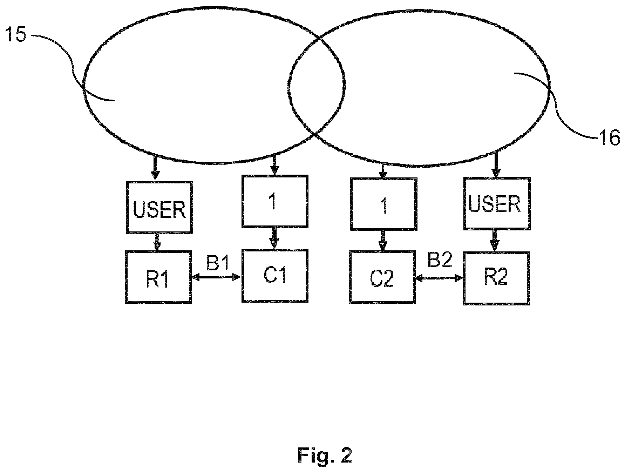

The inventive method allows to assess and to minimize intra-institutional bias between readers (i.e. users) in the same institution, e.g. a clinic or a core lab without additional workload. In the present embodiment illustrated in Fig. 2, two users from the same core lab read studies independently, that is, measure the same anatomical structure of a first patient 15 and of a second patient 16 independently. The first user obtains the first measurement data R1 and the second user obtains the second measurement data R2. The core lab now wants to improve quality in terms of inter-user variability and needs to document that as well. But the lab needs consistency with previous studies, thus an overall drift of measurements cannot be tolerated.

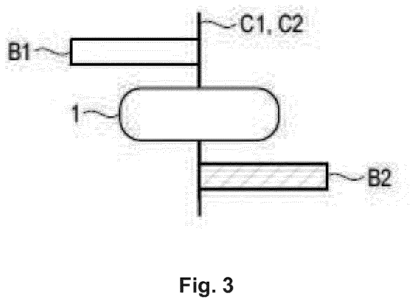

Therefore, an automated measurement tool 1 (baseline machine) reads all studies in parallel. That is, for each manual measurement of the first user and the second user, an automated pendant (i.e. the first baseline measurement data C1 and the second baseline measurement data C2) is performed and stored. In the present embodiment, the baseline measurement data C1, C2 is not displayed to the respective user. Subsequently, for the first user, a first measurement bias B1 based on the first measurement data R1 and the first baseline measurement data C1, and for the second user a second measurement bias B2 based on the second measurement data R2 and second baseline measurement data C2 is determined. In other words, the first and the second measurement bias B1, B2 are determined based on the difference between the first and second measurement data R1, R2 and the first and second baseline measurement data C1, C2, respectively, as illustrated in Fig. 3. In an alternative embodiment the relative biases B1, B2 are determined based on a relative difference of the first and second measurement data R1, R2 and of the first and second measurement baseline data C1, C2. Furthermore, a per-user bias Bx is determined and continuously updated. In the present embodiment a measurement bias B1 for the first user and a measurement bias B2 for the second user.

By comparing the first and the second measurement bias B1, B2, the mean bias between the two users can be calculated. This result can be used to give feedback to all involved users for both a routine clinical echocardiography scenario and a long-term clinical study scenario without the need to measure identical studies. Therefore, the use of the automated measurement tool 1 for determining the measurement bias for each user provides a location independent (i.e. may be used anywhere on earth) reference, similar to the "mètre des Archives" with respect to the length measurement. For the above functionality, the medical image that is chosen by the users can be used to perform the analysis using the automated measurement tool 1 in order to minimize error sources between user and the automated measurement tool 1.

According to a further embodiment of the present invention, by applying the above-mentioned method, an institution-wide bias Bc between all users and the automated measurement tool 1 is determined and continuously updated. Further, a difference $dx = (Bx-Bc)$ can be related to each user. Users with maximum difference in their dx can be chosen for consensus training. Minimizing dx for all users will reduce inter-observer variability without causing an overall drift in the measurements of the core lab. Monitoring the institution-wide bias Bc over time ensures consistent reading behavior of the entire core lab. Monitoring the per-user bias Bx over time ensures consistent reading of each user x - or intended changes due to consensus training. Documentation of all these values can be used to document quality of reading, planning of training and assessing training effects.

According to a further embodiment, the determination of the automated measurement tool 1 is substantially executed in a hidden manner, that is, none of the users is aware of the determination being executed in the background.

Further, the automated measurement tool 1 could also be used to minimize bias between institutions. The automated measurement tool 1 does not necessarily need to be in line with the average way of reading a study in the core lab. That is, the institution wide bias Bc might be large. In other words, the bias might be large without losing its usability as a reference between users and/or between groups of users.

TOMTEC® Arena could be one landing spot of such an invention, but IntelliSpace Cardiovascular (ISCV), which is an image and information management system, would represent a good option as well. Furthermore, such a system could also be integrated in ultrasound systems as communication of the values of the per-user bias Bx would be sufficient to operate it. That is, no transfer of image data would be required.

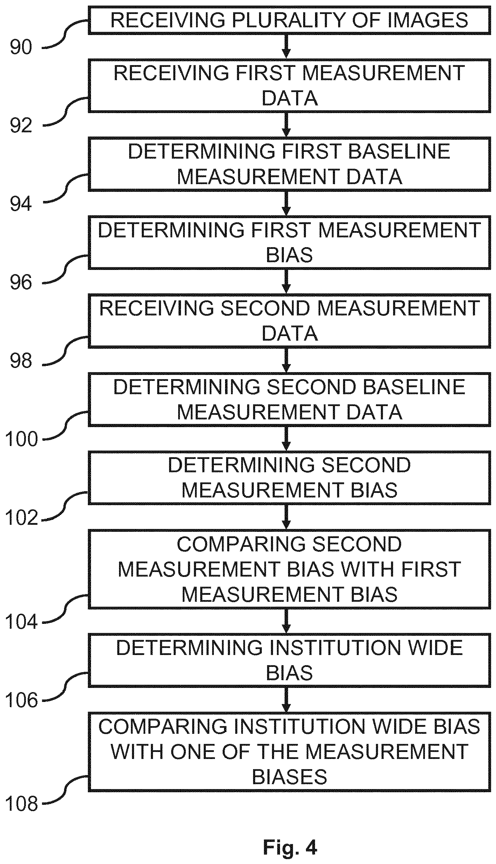

Regarding Fig. 4 the process of the method for automated analysis of the bias in measurements performed on medical images of an anatomical structure will be described in more detail.

First, in step 90 a plurality of medical images is received. The plurality of medical images includes at least a first plurality of medical images and a second plurality of medical images, and each plurality of medical images includes a same anatomical structure of a same or different patient(s). In the present embodiment, the plurality of medical images is not received by the same automated measurement tool 1 but by two automated measurement tools 1. That is, the first plurality of medical images is received by the first automated measurement tool 1 and the second plurality of medical images is received by the second automated measurement tool 1. Further, the automated measurement tools 1 are located at different locations.

Subsequently, in step 92, for the first plurality of medical images, first measurement data R1 of a feature of the anatomical structure is received. The first measurement data R1 is obtained by a first user. Then in step 94, for the first plurality of medical images, a first baseline measurement data C1 of the feature of the anatomical structure is determined by the automated measurement tool 1. Then, in step 96 a first measurement bias B1 for the first user based on the first measurement data R1 and the first baseline measurement data C1 is determined.

Simultaneously or subsequently, in step 98, for the second plurality of medical images, second measurement data R2 of a feature of the anatomical structure is received. The second measurement data R2 is obtained by a second user. The second user being different from the first user. That is, the first and second users are persons different from each other. According to an alternative embodiment the first and second users are the same persons. Then, in step 100, for the second plurality of medical images, a second baseline measurement data C2 of the feature of the anatomical structure is determined by the other automated measurement tool 1. Simultaneously, in step 102, a second measurement bias B2 for the second user based on the second measurement data R2 and the second baseline measurement data C2 is determined.

Finally, in step 104, the first measurement bias B1 is compared with the second measurement bias B2.

According to a further embodiment, the method additionally comprises the step 106 of determining an institution wide bias Bc represented by a mean value of all measurement biases B1, B2,..., Bx of the first, second and further users belonging to one predetermined group or institution after the step 104.

According to a still further embodiment, the method additionally comprises the step 108 of comparing the institution wide bias Bc with one of the measurement biases B1, B2,..., Bx so as to obtain a per-user bias relative to the institution wide bias Bc after the step 106.

According to a further embodiment the step 108 of comparing the institution wide bias Bc with one of the measurement bias B1, B2,..., Bx additionally includes: determining a difference dx1, dx2,..., dxx by subtracting each of the measurement biases B1, B2,..., Bx by the institution wide bias Bc so as to determine the difference dx1, dx2,...,dxx for each user, and determining the user or users having the highest difference(s) dx1, dx2,..., dxx among all users.

In a further embodiment, after step 102, a global bias may be determined represented by a mean value of all available measurement biases Bx. Subsequently, the global bias is compared with one of the measurement biases Bx.

In case an update is performed either of the measurement bias B1, B2,..., Bx of the first, second and further users or of the institution wide bias Bc, the above steps are at least partly repeatedly executed.

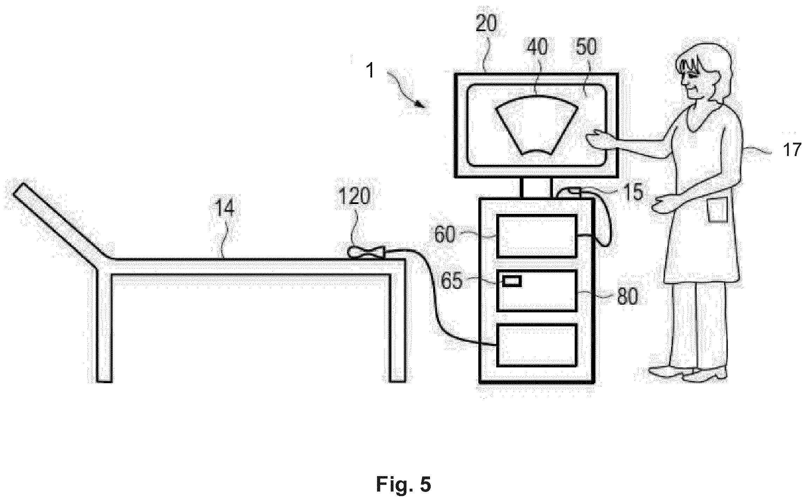

FIG. 5 schematically illustrates an imagining system which in this case is part of an ultrasound imaging machine or ultrasound scanner. The imagining system includes the automated measurement tool 1. Further, the imagining system includes a computer screen 20 having a touch-based display 50. An ultrasound image 40 is currently displayed on the display 50. The imagining system is able to control any images or elements displayed on the display 50. The automated measurement tool lincludes a processor 60, such as a CPU, which is configured to execute an embodiment of the inventive method. The imagining system further includes a data storage medium 80, such as a hard disc, connected to the automated measurement tool 1. Further, the automated measurement tool 1 includes a storage on which a computer program necessary for executing the invention on the automated measurement tool 1 or its processor 60 may be stored. Further, there may be an insertable computer-readable medium 65, e.g. USB-stick, which may be used to load the necessary computer program onto the automated measurement tool 1. Finally, the imagining system may include an ultrasound control unit 200, which controls the acquisition of ultrasound images by an ultrasound probe 120. A patient bed 14 is provided for a subject, e.g. a patient, reclining while ultrasound images 40 are being acquired by the user 17. The user 17 may be any person who wishes to perform an accurate image evaluation, which in the case of medical images will often be a radiologist or radiographer, but can also be a specialist in any other field, such as gynaecologist, a cardiologist etc.

While the invention has been illustrated and described in detail in the drawings and foregoing description, such illustration and description are to be considered illustrative or exemplary and not restrictive; the invention is not limited to the disclosed embodiments.

## Claims

1. A computer implemented method for automated analysis of the bias in measurements performed on medical images of an anatomical structure, wherein the method comprises the steps of:

   receiving a plurality of medical images, wherein the plurality of medical images includes at least a first plurality of medical images and a second plurality of medical images, and each plurality of medical images includes the same anatomical structure of the same or different patient(s),
   receiving, for the first plurality of medical images, first measurement data (R1) of a feature of the anatomical structure, wherein the first measurement data (R1) is obtained by a first user,
   determining, for the first plurality of medical images, a first baseline measurement data (C1) of the feature of the anatomical structure by means of an automated measurement tool (1),
   determining a first measurement bias (B1) for the first user based on the first measurement data (R1) and the first baseline measurement data (C1),
   receiving, for the second plurality of medical images, second measurement data (R2) of a feature of the anatomical structure, wherein the second measurement data (R2) is obtained by a second user, the second user being different from the first user,
   determining, for the second plurality of medical images, a second baseline measurement data (C2) of the feature of the anatomical structure by means of an automated measurement tool (1),
   determining a second measurement bias (B2)

for the second user based on the second measurement data (R2) and the second baseline measurement data (C2), and
comparing the first measurement bias (B1) with the second measurement bias (B2).

2. Method for automated analysis of the bias in measurements performed on medical images of an anatomical structure according to claim 1, wherein the method further comprises the step of
determining a global bias represented by a mean value of all available measurement biases (B1, B2,..., Bx), and
comparing the global bias with one of the measurement biases (B1, B2,..., Bx).

3. Method for automated analysis of the bias in measurements performed on medical images of an anatomical structure according to claim 1 or 2,
wherein the first measurement data (R1) is determined based on one specific medical image out of the first plurality of medical images and the first baseline measurement data (C1) is determined based on the same specific medical image, and
wherein the second measurement data (R2) is determined based on one specific medical image out of the second plurality of medical images and the second baseline measurement data (C2) is determined based on the same specific medical image.

4. Method for automated analysis of the bias in measurements performed on medical images of an anatomical structure according to any one of claims 1 to 3, wherein the measurement bias (B1, B2,..., Bx) is continuously updated once new measurement data (R1, R2,..., Rx) is available,
wherein all available measurement data (R1, R2,..., Rx) are used to update the measurement bias (B1, B2,..., Bx), or
wherein all available measurement data (R1, R2,..., Rx) obtained during a pre-determined past period of time are used to update the measurement bias (B1, B2,..., Bx).

5. Method for automated analysis of the bias in measurements performed on medical images of an anatomical structure according to any one of claims 1 to 4, wherein the method further comprises the step of:
determining an institution wide bias (Bc) represented by a mean value of all measurement biases (B1, B2,..., Bx) of the first, second and further users belonging to one predetermined group or institution.

6. Method for automated analysis of the bias in measurements performed on medical images of an anatomical structure according to claim 5, wherein the method further comprises the step of:
comparing the institution wide bias (Bc) with one of the measurement biases (B1, B2,...,Bx) so as to obtain a per-user bias relative to the institution wide bias (Bc).

7. Method for automated analysis of the bias in measurements performed on medical images of an anatomical structure according to claim 6, wherein the step of comparing the institution wide bias (Bc) with one of the measurement bias (B1, B2,..., Bx) includes:

    determining a difference (dx1, dx2,..., dxx) by subtracting each of the measurement biases (B1, B2,..., Bx) by the institution wide bias (Bc) so as to determine the difference (dx1, dx2,..., dxx) for each user belonging to the same group or institution, and
    determining the user or users having the highest difference(s) (dx1, dx2,..., dxx) among all users.

8. Method for automated analysis of the bias in measurements performed on medical images of an anatomical structure according to any one of claims claim 5 to 7, wherein the determination of at least the first baseline measurement data (C1) and the second baseline measurement data (C2) takes place at different locations.

9. Method for automated analysis of the bias in measurements performed on medical images of an anatomical structure according to any one of claims 1 to 8, wherein the automated measurement tool uses artificial intelligence for determining the baseline measurement data (C1, C2,..., Cx).

10. Method for automated analysis of the bias in measurements performed on medical images of an anatomical structure according to any one of claims 1 to 9, wherein the automated measurement tool uses machine learning for determining the baseline measurement data (C1, C2,..., Cx), wherein the automated measurement tool is trained by a reference data base including reference images of the anatomical structure and measurement data obtained by at least one expert user for said reference medical images.

11. Method for automated analysis of the bias in measurements performed on medical images of an anatomical structure according to any one of claims 1 to 10, wherein the automated measurement tool uses an artificial neural network for determining the baseline measurement data (C1,C2,...,Cx).

12. Method for automated analysis of the bias in measurements performed on medical images of an anatomical structure according to any one of claims 1 to 11, wherein the measurement data includes at least one of a dimension such as a length, volume, area, volume flow, velocity of blood and velocity of tissue.

13. Method for automated analysis of the bias in measurements performed on medical images of an anatomical structure according to any one of claims 1 to 12,

wherein for the measurement bias (B1, B2, ..., Bx), a new measurement bias ($B1_{new}$, $B2_{new}$,..., $Bx_{new}$) and an old measurement bias ($B1_{old}$, $B2_{old}$,..., $Bx_{old}$) are determined,

wherein the new measurement bias ($B1_{new}$, $B2_{new}$,..., $Bx_{new}$) is determined when a predetermined period of time has elapsed since the old measurement bias ($B1_{old}$, $B2_{old}$,..., $Bx_{old}$) was determined, or the new measurement bias ($B1_{new}$, $B2_{new}$,..., $Bx_{new}$) is determined when a predetermined amount of new measurement data (R1, R2, ..., Rx) are available since the old measurement bias ($B1_{old}$, $B2_{old}$,..., $Bx_{old}$) was determined, and

wherein the new measurement bias ($B1_{new}$, $B2_{new}$,..., $Bx_{new}$) is compared with the old measurement bias ($B1_{old}$, $B2_{old}$,..., $Bx_{old}$) so as to obtain a drift in the measurement bias (B1, B2, ..., Bx).

14. Computer program comprising program code instructions, which, when executed by a processor, enables the processor to carry out the method according to any one of claims 1 to 13.

15. An automated measurement tool (1) configured to execute the method according to any one of claims 1 to 13, wherein the automated measurement tool (1) includes an interface configured to receive a medical image generated by an imaging system (10) and to output results determined by executing the method.

Fig. 1

**Fig. 2**

Fig. 3

RECEIVING PLURALITY OF IMAGES

90

RECEIVING FIRST MEASUREMENT DATA

92

DETERMINING FIRST BASELINE MEASUREMENT DATA

94

DETERMINING FIRST MEASUREMENT BIAS

96

RECEIVING SECOND MEASUREMENT DATA

98

DETERMINING SECOND BASELINE MEASUREMENT DATA

100

DETERMINING SECOND MEASUREMENT BIAS

102

COMPARING SECOND MEASUREMENT BIAS WITH FIRST MEASUREMENT BIAS

104

DETERMINING INSTITUTION WIDE BIAS

106

COMPARING INSTITUTION WIDE BIAS WITH ONE OF THE MEASUREMENT BIASES

108

**Fig. 4**

**Fig. 5**

Europäisches
Patentamt
European
Patent Office
Office européen
des brevets

## EUROPEAN SEARCH REPORT

Application Number

EP 19 20 2004

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Man against machine: AI is better than dermatologists at diagnosing skin cancer", , 28 May 2018 (2018-05-28), XP055667632, Retrieved from the Internet: URL:https://eurekalert.org/pub_releases/2018-05/esfm-mam052418.php [retrieved on 2020-02-12] * the whole document * ----- | 1-5,9-15 | INV. G06T7/00 |
| A | ZORAN B. POPOVIC ET AL: "Assessing observer variability: a user's guide", CARDIOVASCULAR DIAGNOSIS AND THERAPY, vol. 7, no. 3, June 2017 (2017-06), pages 317-324, XP055647283, ISSN: 2223-3652, DOI: 10.21037/cdt.2017.03.12 * the whole document * ----- | 1-15 | |
| | | | TECHNICAL FIELDS SEARCHED (IPC) G06T |

The present search report has been drawn up for all claims

| Place of search | Date of completion of the search | Examiner |
|---|---|---|
| Munich | 12 February 2020 | Rockinger, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2018150598 A1

- US 2014341449 A1

### Non-patent literature cited in the description

- Fully Automated Versus Standard Tracking of Left Ventricular Ejection Fraction. *JOURNAL OF THE AMERICAN COLLEGE OF CARDIOLOGY,* vol. 66 (13), 2015
- **VIKRAM CHALANA.** A Methodology for Evaluation of Boundary Detection Algorithms on Medical Images. *IEEE TRANSACTIONS ON MEDICAL IMAGING,* October 1997, vol. 16 (5
- **HAROLD L. KUNDE.** Measurement of Observer Agreement. *Radiology,* 2003, vol. 228, 303-308

- **LANG.** The American Society of Echocardiography Recommendations for cardiac chamber Quantification in Adults: A Quick Reference Guide from the Ase Workflow and Lab Management Task Force", in "Recommendations for Cardiac Chamber Quantification by Echocardiography in Adults: An Update from the American Society of Echocardiography and the European Association of Cardiovascular Imaging. *Journal of the American Society of Echocardiography,* vol. 28
- **NAGUEH.** Recommendations for the Evaluation of Left Ventricular Diastolic Function by Echocardiography: An Update from the American Society of Echocardiography and the European Association of Cardiovascular Imaging. *Journal of the American Society of Echocardiography,* April 2016